# EUROPEAN PATENT SPECIFICATION

(11) **EP 3 188 681 B1**
(45) Date of publication and mention of the grant of the patent: **27.06.2018**
(21) Application number: 15781153.0
(22) Date of filing: 02.09.2015
(51) Int. Cl.: A61B 18/26, A61B 17/22

(54) **DEVICE FOR FRAGMENTING ORGANO-MINERAL CONCRETION**
VORRICHTUNG ZUR ZERKLEINERUNG VON MINERALORGANISCHEM BETON
DISPOSITIF POUR LA FRAGMENTATION D'UNE CONCRÉTION ORGANO-MINÉRALE

(30) Priority: 02.09.2014 US 201462044573 P
(43) Date of publication of application: 12.07.2017
(73) Proprietor: Nordson Corporation, Westlake, OH 44145 (US)
(72) Inventor: DIAMANT, Valery, 12900 Katzrin (IL); CHEPOVETSKY, Gennady, 2012100 Halutz (IL); EREMENKO, Sergey, 299018 Sevastopol (RU); EREMENKO, Alexey, 350030 Krasnodar (RU); MARTOV, Alexey, 127434 Moscow (RU); GUDKOV, Alexander, 634026 Tomsk (RU); LERNER, Marat, 634041 Tomsk (RU); CHERNENKO, Vladimir, 634055 Tomsk (RU)
(74) Representative: Zacco GmbH
(86) International application number: PCT/IL2015/050878
(87) International publication number: WO 2016/035071

(56) References cited:
- US-A- 5 246 447
- US-A- 5 425 735
- US-B1- 6 352 535

## Description

### FIELD OF THE INVENTION

This invention relates to a device for fragmenting abnormal concretions in body cavities, and in particular, to a medical technique for fragmenting hard solid calculous formations in the ducts and cavities of a living body.

### BACKGROUND OF THE INVENTION

Due to numerous reasons, all manner of organo-mineral concretions can be formed in the cavities of organs of humans and other mammals. Occlusions of various blood vessels, including calcified vessels, salivary gland calculi, urinary system calculi, biliary calculi, and so forth are examples of such concretions. Disintegration of such concretions is a highly important issue today.

Several techniques are employed in clinical practice for breaking abnormal concretions appearing in the biliary and/or urinary system of a human body into pieces for further removal of the pieces from the body. To cure the most complicated forms of nephroureterolithiasis (large, multiple, and coralloid renal calculi, "impacted" and large ureteral calculi, etc.), endourological methods are increasingly used. In particular, the use of percutaneous and transurethral contact lithotripsy makes it possible to reduce perioperative risks of remote lithotripsy and open lithotomy, as well as to reduce the duration of inpatient and outpatient treatment.

Although procedures have varied, most of them have involved dilatating, anesthetizing and lubricating the urinary or biliary tract, and then attempting to grasp the calculus for crushing it, and then dragging it out.

The principal methods in current use for intracorporeal lithotripsy are the ultrasonic, pneumatic, electrokinetic, laser, and electrohydraulic methods. For example, intra-corporeal shock wave lithotripsy employs high-energy shock waves to fragment and disintegrate calculi. Ultrasonic lithotripsy technique is known that utilizes an ultrasound probe emitting high-frequency ultrasonic energy towards a concretion. For this technique, direct contact of the probe tip and stone is essential for effectiveness of ultrasonic lithotripsy.

Each method has advantages and drawbacks. For instance, in applying ultrasonic lithotripsy, only rigid probes and rigid endoscopes can be used, and the scope of its application is currently limited primarily to renal calculi. Impact lithotripsy (pneumatic or electrokinetic methods) is one of the most effective and safe methods for contact fragmentation of stones. Use of such lithotripters is also restricted to rigid endoscopes, and retrograde calculus propulsion during transurethral ureterolithotripsy is regarded as a drawback of this method. The electrohydraulic lithotripsy (EHL) and laser lithotripsy methods, being effective methods for contact crushing, can be used with both rigid and flexible endoscopes, which substantially expand the scope of their application in modern urology.

The EHL technique is effective in breaking urinary stones into pieces small enough for basket extraction or simple passage. When EHL is selected to affect destruction of the stone, the EHL probe is placed in proximity to the stone. By means of an electrical discharge, a shock wave is produced which impacts the surface of the stone and produces tiny cracks. When enough cracks have been made, the stone shatters into small pieces. The individual pieces can then be attacked one at a time, or they can further be removed by basket extraction. However, the electrohydraulic method gives rise to a much greater number of complications as compared to other methods, since the produced shock wave damages the surrounding tissues when the discharge occurs too close to the urinary tract walls.

On the other hand, laser based technologies are widely used today in medicine for various purposes, including fragmentation various types of organo-mineral concretions. Lasers are known as an alternative source of energy in lithotripsy, especially for the destruction of renal stones. Various types of laser lithotripsy probes with a variety of laser sources, including pulsed dye laser, alexandrite laser, neodymium laser, holmium laser and other lasers, have been developed. Laser fragmentation is safer than EHL, but it takes longer for fragmentation to occur, and laser lithotripsy requires more costly equipment. Furthermore, frequent damage to the flexible ureteropyeloscope, which occurs due to breakage of the laser fiber inside the bent endoscope, is a major drawback of this technique.

A lithotripsy technique of nanosecond electro-pulse destruction of materials is also known in the art (see, for example, U.S. Pat. No. 7,087,061 and U.S. Pat. Publ. No. US 2007/0021754) that can be used for safe contact fragmentation of calculi in all parts of a patient's urinary tracts. This technique employs probes of various diameters, compatible with both rigid and flexible endoscopes.

The nanosecond electric breakdown carried out through the calculus results in the rise of a plasma channel, occurrence of micro-explosions, and the emergence of numerous thermo-mechanical tensile stresses in the concretions. However, due to its operating principle, the nanosecond electro-pulse lithotripsy (NPL) is essentially different from the electrohydraulic lithotripsy (EHL). Contrary to electrohydraulic destruction, employing electrodes which are not in direct contact with the object, electro-pulse destruction utilizes a probe with electrodes which are placed directly on the object's surface, and uses short, nanosecond electric pulses with steep fronts to fragment stones. This technique is based on the Vorob'evs effect that provides certain features of the discharge observed when a solid dielectric in contact with two rodlike electrodes is placed in a liquid dielectric medium, and a voltage pulse with increasing front is applied to the electrodes. According to this effect, when the pulse front slope is not so steep (e.g., the pulse rise time is more than about 0.3 microseconds), a discharge develops in the surrounding liquid over the solid dielectric surface, rather than penetrating into the solid body. On the other hand, in the case of a sufficiently steep slope of the pulse front (e.g., the pulse rise time is less than about 100 ns), the discharge current propagates through the solid. This gives rise to tensile mechanical effects in the calculus, which causes its cracking and, finally, fragmentation (see, for example, G.A. Masyats, Technical Physics Letters, Vol. 31, No. 12, 2005, pp. 1061-1064. Translated from Russian from Pis'ma v Zhurnal Tekhnicheskoi Fiziki, Vol. 31, No. 24, 2005, pp. 51-59).

Shock waves during application of electrohydraulic lithotripsy (EHL) technique may result in serious damage to surrounding tissues and for this reason the EHL technique virtually ceased being in use for endoscopic fragmentation. On the other hand, when nanosecond electro-pulse lithotripsy (NPL) technique is used for fragmentation of calculi or other concretions, the energy of the electric pulse is released directly within the bulk of the solid body being fragmented, rather than in the surrounding liquid. Thus, the NPL technique requires considerably less energy for disintegration of concretions than the EHL technique, and makes the method safer.

Lasers used in medicine offer a wide range of wavelengths from the ultraviolet band to the near infrared band, including the visible part of the spectrum. Primary lasers currently in use are the Holmium, Ho:YAG laser with a wavelength of 2.140 µm, the Neodym, Nd:YAG laser with a wavelength of 1.064 µm, the Kalium titanyl phosphate KTP:Nd:YAG (SHG) laser with a wavelength of 0.532 µm, the Lithium borat LBO:Nd:YAG (SHG) laser with a wavelength of 0.532 µm, the Thulium Tm:YAG laser with a wavelength of 2.013 µm, Dye Lasers with wavelengths in a wide range, CO - lasers with a wavelength of 10.6 µm, and Diode lasers with wavelengths of 0.830 µm, 0.940 µm, 0.980 µm, 1.318 µm, and 1.470 µm.

Laser methods for fragmentation of organo-mineral concretions are well-known and are widely used nowadays. An example of such use can be the Ho: YAG laser with a wavelength of 2.1 µm, employed in urology for the fragmentation of urological stones. The interaction of laser radiation with the surface of tissues and concretions, or the fragmentation of organo-mineral concretions by using lasers is related to a combination of three principal mechanisms: photothermal, photomechanical, and the cavitation bubble effect, that is to say, a shockwave mechanism. The photothermal and photomechanical effects are related to the direct absorption of laser energy by the body being irradiated. Molecules of liquid contained in a body, be it one composed of biological tissues or organo-mineral concretions, absorb a certain wavelength of laser light, which results in their evaporation. Moreover, the high temperature caused by the energy of the laser pulse has the effect of destabilizing the chemical structure of organo-mineral concretions, such that the laser creates a crater on the surface of the body being irradiated. In addition, the laser beam forms spherical cavitation bubbles, which then produce a shock wave as a result of their collapse. This shock wave produces a photoacoustical effect that also promotes fragmentation of organo-mineral concretions. Therefore, a phenomenon may here be observed which is similar to that observed during electrohydraulic lithotripsy. In cases where laser radiation directly affects biological tissues, there occurs a thermic effect whose depth depends on the laser wavelength, which is related to the laser's coefficient of absorption by water, tissues, etc.

Investigations of the Applicant have demonstrated that NPL is the most effective method for destroying biological concretions as compared to the laser or electrohydraulic method.

However, the effectiveness of this method depends fundamentally on the physical properties of the concretion, such as its density and structure. As the density of a concretion rises, the effectiveness of NPL is reduced (see for example, Alexey Martov, Valery Diamant, Artem Borisik, Andrey Andronov, and Vladimir Chernenko. Comparative in Vitro Study of the Effectiveness of Nanosecond Electrical Pulse and Laser Lithotripters. JOURNAL OF ENDOUROLOGY, 2013, V. 27, N. 10, P. 1287 - 1296; Alexey Martov, Alexander Gudkov, Valery Diamant, Gennady Chepovetsky, and Marat Lerner. Investigation of Differences between Nanosecond Electro-pulse and Electrohydraulic Methods of Lithotripsy: A Comparative In Vitro Study of Efficacy. JOURNAL OF ENDOUROLOGY, 2014, V. 28, N. 4, P. 437 - 445. Reference is also made to document US 5425735 A.

### SUMMARY OF THE INVENTION

As described above, laser techniques allow practically any calculus to be fragmented, but the efficiency of such a laser lithotripter is not high, because a considerable amount of time is required to fragment even a small stone.

On the other hand, nanosecond electro-pulse lithotripsy (NPL) is a highly effective and relatively safe technique that allows for contact fragmentation of organo-mineral concretions in all parts of the urinary tract, as well as in other hollow cavities of organs, such as blood vessels, biliary ducts, etc. Investigation of the Applicants revealed that this technique is much more effective for the fragmentation of concretions than other methods in use today, such as the laser and EHL techniques. However, the efficacy of NPL, as well as some other methods of contact lithotripsy, depends on the density and structure of organo-mineral concretions. Thus, there is dependence between the properties of organo-mineral concretions and the cumulative energy required for their fragmentation.

At the same time, when the surface of organo-mineral concretions is irradiated by laser radiation of some wavelengths, such radiation may change the surface properties of the organo-mineral concretions. It was found by the Applicants that even if this laser radiation has low energy, being insufficient to fragment concretions, the defects which can be created in the concretions may essentially affect the efficacy of NPL, if this technique is used after laser irradiation.

Thus, there is a need in the art for, and it would be useful to have a novel medical device and method for intracorporeal lithotripsy that provide fast and easy fragmentation of any organo-mineral concretions and their pieces inside hollow cavities of organs. In addition, it would be advantageous to reduce the procedure time and lessen the likelihood of injury to adjacent body tissues during treatment.

The term "concretion" as used herein refers to solid calculous formations of urates, oxalates and phosphates, e.g. gallstones, kidney stones, cystine stones and other calculi, lodged in the ducts and cavities of a living body.

It would be advantageous to combine the effects of laser lithotripsy and NPL in a device which is capable to be safely introduced into the confined space of the individual ureter, urinary bladder or biliary tract, blood vessels etc. and create cracks and/or shatter the concretion into smaller pieces.

It would be useful to have a method that can provide fragmentation of organo-mineral concretions due to applying NPL together with the preliminary effect of laser radiation on the concretion. The specified method can open up new horizons for fast fragmentation of large and dense organo-mineral concretions, for example, large dense stones or staghorn stones in the urinary tract, when used in clinical practice using retrograde access. The invention is defined in the appended set of claims. Methods mentioned hereinafter do not form part of the invention. The present invention satisfies the aforementioned need by providing a novel medical device for breaking a concretion in a body into smaller pieces that allows a user to exert the combined effect of laser radiation and NPL onto organo-mineral concretions, so they are fragmented more rapidly and efficiently, when compared with the use of laser radiation and NPL separately.

The medical device includes a combined probe including a laser waveguide probe and a nanosecond electro-pulse lithotripter probe.

The medical device also includes an optical energy source coupled to the laser waveguide probe. The optical energy source is configured to generate a laser radiation field having energy sufficient to form a defect on a surface of the organo-mineral concretion when the laser waveguide probe is applied to the organo-mineral concretion.

The medical device also includes an electrical energy source coupled to the nanosecond electro-pulse lithotripter probe. The electrical energy source is configured to generate high-voltage nanosecond electro-pulses having energy sufficient to break the organo-mineral concretion by providing a spark electrical discharge through the organo-mineral concretion when the nanosecond electro-pulse lithotripter probe is applied to the organo-mineral concretion.

The medical device also includes a monitoring and control system configured for monitoring operation parameters and controlling operation of the device by switching operation of the device from activating of the laser waveguide probe for generating laser radiation, to activating of the nanosecond electro-pulse lithotripter probe for generating nanosecond electric pulses.

According to an embodiment, the laser waveguide probe includes one or more laser fibers for providing laser radiation to the organo-mineral concretion. Likewise, the nanosecond electro-pulse lithotripter probe includes an operating head configured to provide spark electrical discharge through the organo-mineral concretion.

According to an embodiment, a distal portion of the laser fiber of the laser waveguide probe is arranged coaxially with the operating head of the nanosecond electro-pulse lithotripter probe. For example, the laser fiber is arranged along the longitudinal axis of the combined probe. The operating head nanosecond electro-pulse lithotripter probe includes lithotripter electrodes formed as concentrically placed tubular bushings surrounding the laser fiber. Alternatively, the laser fiber has a tubular shape, and the operating head is arranged within the laser fiber.

According to an embodiment, the laser fiber of the laser waveguide probe is arranged in parallel relation to the operating head of the nanosecond electro-pulse lithotripter probe.

According to an embodiment, the combined probe includes an external sheath surrounding the laser waveguide probe and a nanosecond electro-pulse lithotripter probe.

According to an embodiment, the combined probe includes a manipulator configured to move the laser fiber and the operation head of the electro-pulse lithotripter probe independently of one another for bringing them into direct contact with the concretion.

The present invention may be employed in a method for destruction of the concretion utilizing the device of the present invention. The method includes generating a laser radiation field having energy sufficient to form a defect on the surface of the organo-mineral concretion; generating high-voltage nanosecond electric pulses having energy sufficient to break the organo-mineral concretion by providing the spark electrical discharge through the organo-mineral concretion; applying the laser waveguide probe to the surface of the concretion, and treating the surface with a laser radiation field to create a defect; and applying the nanosecond electro-pulse lithotripter probe to the treated surface to provide a spark electrical discharge through the concretion.

Accordingly, the method also includes manipulating the laser fiber for bringing it to a surface of the concretion for irradiating with a laser radiation field to create defects on the surface. The method also includes manipulating at least one of the electrodes of the operation head of the electro-pulse lithotripter probe for bringing the electrode into direct contact with the concretion in order to form an electrical spark with a discharge channel that is capable of generating shock waves and providing stresses that exceed the strength of the concretion material.

The use of laser radiation in the method employing the present invention can form one or more defects onto the organo-mineral concretion surface that reduce the breakdown voltage provided by the nanosecond electro-pulse lithotripter (NPL) probe when the NPL probe is applied after the laser treatment, and also can increase the NPL efficacy.

Thus, the method provides destruction of the concretion by performing intra-corporeal lithotripsy through sequential treatment of a concretion with laser irradiation and then further shattering it into fragments with nanosecond electric pulses that generate substantial tensile stresses, leading to fragmentation of the concretion.

According to an embodiment of the present invention, the range of laser wavelengths lies within 0.94 µm - 10.6 µm.

According to an embodiment of the present invention, the total cumulative energy during laser surface treatment lies within the range of a few Joules to several thousand Joules. Preferably, the total cumulative energy during laser surface treatment lies within the range of 15 Joules to 250 Joules.

According to an embodiment of the present invention, the surface is treated by continuous laser radiation.

According to an embodiment of the present invention, the surface is treated by pulsed laser radiation. The pulses of the pulsed laser radiation can have durations of 0.1 to 60 ms, a pulse frequency ranging from 1 to 30 Hz, and power within 0.5 to 40 W. According to an embodiment of the present invention, the energy in the laser pulse is in the range of 0.3 Joule to 5 Joules.

According to an embodiment of the present invention, the energy in the pulse of the electro-pulse lithotripter, applied to the concretion following laser treatment, is in the range of 0.1 Joule to 2 Joules.

For example, just a single electric pulse can be used. Alternatively, a train of electric pulses can be used which are applied at a frequency in the range of 1 Hz to 20 Hz.

The surface of the organo-mineral concretion can be treated with a laser and a nanosecond electro-pulse probe sequentially multiple times until total disintegration of the concretion. For example, after the initial laser treatment of the surface of the concretion to be fragmented, and its fragmentation into several parts by the nanosecond electro-pulse treatment, each fragment is treated with a laser and then fragmented into smaller fragments using the nanosecond electro-pulse lithotripter and so on, until the concretion has completely disintegrated.

There has thus been outlined, rather broadly, the more important features of the invention in order that the detailed description thereof that follows hereinafter may be better understood. Additional details and advantages of the invention will be set forth in the detailed description, and in part will be appreciated from the description, or may be learned by practice of the invention.

### BRIEF DESCRIPTION OF THE DRAWINGS

In order to understand the invention and to see how it may be carried out in practice, preferred embodiments will now be described, by way of non-limiting example only, with reference to the accompanying drawings, in which:
**Fig. 1** is a schematic block diagram of the device combining electro-pulse fragmentation of organo-mineral concretions and a laser treatment of a concretion surface, according to one embodiment of the present invention;
**Fig. 2** illustrates a schematic exterior view of the device for breaking a concretion in a body into smaller pieces, according to one embodiment of the present invention;
**Fig. 3A** illustrates a schematic side partially cross-sectional view of a distal portion of a probe of the medical device for breaking a concretion in a body into smaller pieces, according to one embodiment of the present invention;
**Fig. 3B** illustrates a schematic enlarged side cross-sectional view of the distal portion of the probe the medical device of Fig. 3A;
**Fig. 3C** illustrates a schematic transverse cross-sectional view of the distal portion of the medical device of Fig. 3A taken along the line A - A;
**Fig. 3D** illustrates a schematic enlarged side cross-sectional view of the distal portion of the probe the medical device according to another embodiment of the present invention;
**Fig. 4A** illustrates a schematic side partially cross-sectional view of a distal portion of the probe of the medical device for breaking a concretion in a body into smaller pieces, according to still another embodiment of the present invention;
**Fig. 4B** illustrates a schematic enlarged side cross-sectional view of the distal portion of the probe the medical device of Fig. 4A;
**Fig. 4C** illustrates a schematic transverse cross-sectional view of the head of the medical device of Fig. 4A taken along the line B - B;
**Fig. 5A** illustrates a schematic side partially cross-sectional view of a distal portion of the probe of the medical device for breaking a concretion in a body into smaller pieces, according to a further embodiment of the present invention;
**Fig. 5B** illustrates a schematic enlarged side cross-sectional view of the distal portion of the probe the medical device of Fig. 5A;
**Fig. 5C** illustrates a schematic transverse cross-sectional view of the probe head of the medical device of Fig. 5A taken along the line C - C;
**Fig. 6** shows an averaged total specific volumetric energy required to break experimental samples by using various techniques;
**Fig. 7** shows the dependence of specific energy required to fragment concretions for nanosecond electro-pulse treatment versus stone density and probe diameter; and
**Figs. 8A and 8B** show experimental data for the cumulative energy required for fragmentation of a calculus when only a nanosecond electro-pulse treatment was applied, and when a nanosecond electro-pulse treatment was applied after the preliminary laser treatment for various types of laser and dimensions of the nanosecond electro-pulse probe.

### DETAILED DESCRIPTION OF EXEMPLARY EMBODIMENTS

The principles of the method for the medical device according to the present invention may be better understood with reference to the drawings and the accompanying description, wherein like reference numerals have been used throughout to designate identical elements. It is understood that these drawings are not necessarily to scale, are given for illustrative purposes only, and are not intended to limit the scope of the invention. Examples of constructions, materials, dimensions, and manufacturing processes are provided for selected elements. Those versed in the art should appreciate that many of the examples provided have suitable alternatives which may be utilized.

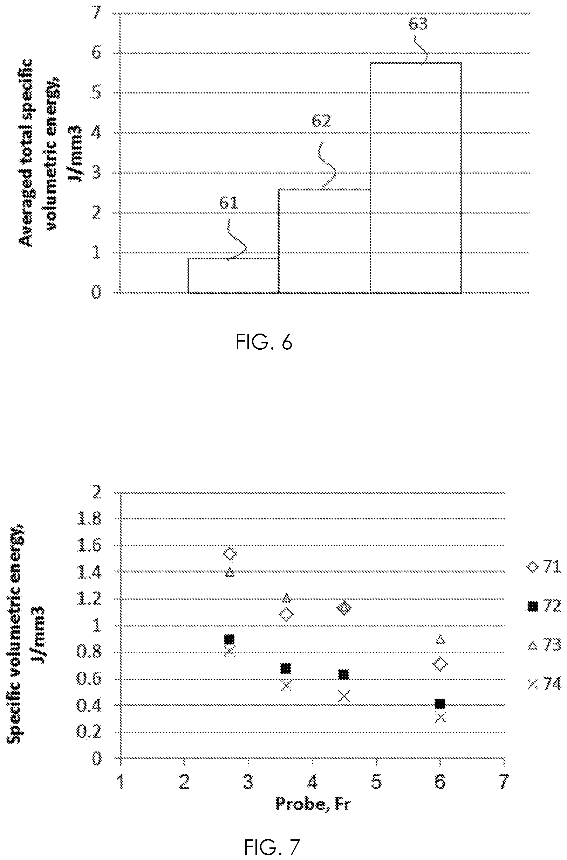

The inventors of the present application have demonstrated that NPL is the most effective method for destroying biological concretions as compared to the laser or electrohydraulic method. For example, **Fig. 6** shows an averaged total specific volumetric energy required to break experimental samples of organo-mineral concretions for the following techniques: NPL - nanosecond electro-pulse lithotripter technique (indicated by a reference numeral **61**), EHL - electrohydraulic lithotripter technique (indicated by a reference numeral **62**); and LL - Ho:YAG laser lithotripter technique (indicated by a reference numeral **63**). As can be seen, the specific volumetric energy required for destroying concretions is less than 1 J/mm³ for the NPL technique. This energy is greater than 2 J/mm³ for the EHL technique and greater than 5 J/mm³ for the laser lithotripter technique.

Moreover, it was also found that the effectiveness of the NPL method depends not only on the density and structure of the concretion, but also on the dimension of the NPL probe. **Fig. 7** shows the dependence of specific energy required to fragment concretions for the NPL technique versus stone density and probe diameter. Electrical pulses of energy of 1 Joule and 0.8 Joule were used when NPL was used for destruction of soft and hard organo-mineral concretions. Symbols indicated by a reference numeral **71** correspond to the experiments for destruction of hard concretions using electrical pulses of energy of 1 Joule. Symbols indicated by a reference numeral **72** correspond to the experiments for destruction of soft concretions using electrical pulses of energy of 1 Joule. Symbols indicated by a reference numeral **73** correspond to the experiments for destruction of hard concretions using electrical pulses of energy of 0.8 Joule. Symbols indicated by a reference numeral **74** correspond to the experiments for destruction of soft concretions using electrical pulses of energy of 0.8 Joule.

Probes having diameters of 4.5 Fr (French) and 6 Fr were used in the experiments.

As can be seen in **Fig. 7**, while working with probes having (i.e. 3.6 Fr), which are typically used for managing blood vessels, kidney stones, etc., as well as when stone density is increased, fragmentation energy and time are relatively greater than when working with probes having large diameter (i.e. 6 Fr). This is an undesirable effect, which complicates the procedure and may lead to tissue damage due to the increase in fragmentation energy required to achieve a greater effect for probes with small diameters.

In this connection, the inventors of the present application contemplated a method that allows increasing efficacy of the NPL technique. Thus, according to an embodiment of the present invention, at the first stage, a surface of the concretions is irradiated by a laser beam, in order to create defects on the surface. After treatment with laser radiation, fragmentation of the concretion can be culminated by applying the nanosecond electro-pulse lithotripsy technique, that provides a spark discharge through the concretion, resulting in destruction of the concretion into small pieces. Such a combined treatment can provide the possibility to fragment even large and dense concretions by applying insignificant cumulative energy for a short time period.

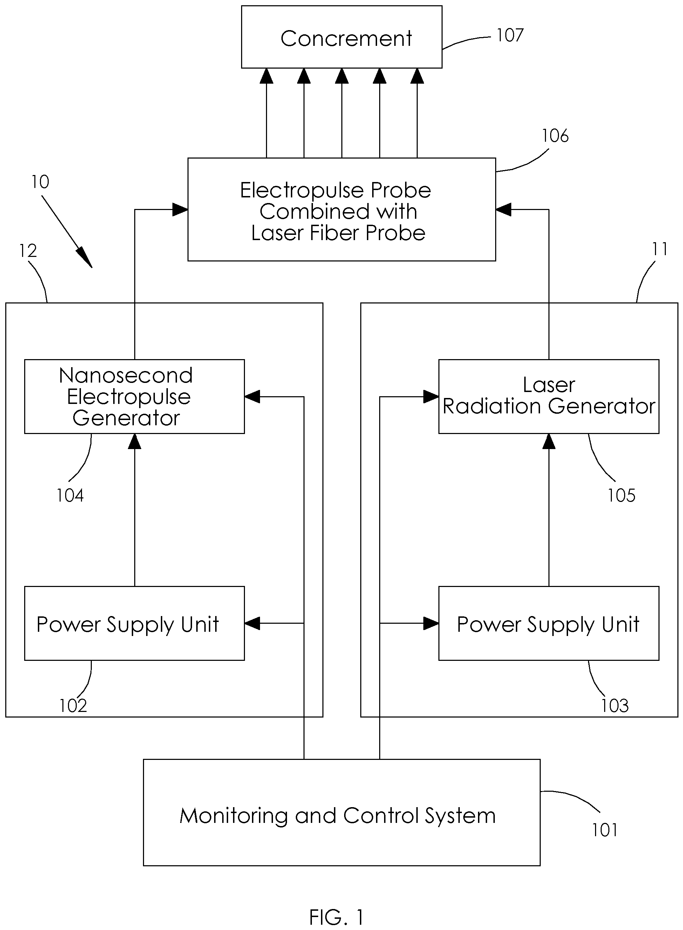

**Fig. 1** illustrates a schematic block diagram of a device **10** for breaking an organo-mineral concretion **107** into smaller pieces by combining a laser treatment and electro-pulse breakdown techniques, according to one embodiment of the present invention. The device for breaking a concretion into smaller pieces includes a probe **106** which has two parts, such as a laser waveguide probe (not shown in **Fig. 1**), which has one or more laser fibers, and a nanosecond electro-pulse lithotripter probe (not shown in **Fig. 1**) which has electrodes.

Since the probe **106** includes both a laser waveguide probe and a nanosecond electro-pulse lithotripter probe, it is also referred to as a "combined probe".

The combined probe **106** is coupled to an optical energy source **11** including a laser radiation generator **105** optically coupled to the laser waveguide probe and configured for generating laser radiation. The combined probe **106** is also coupled to an electrical energy source **12** including a nanosecond electro-pulse generator **104** electrically coupled to the nanosecond electro-pulse lithotripter probe and configured for generating high-voltage pulses. According to the present invention, laser radiation is required to form defects on the surface of the concretion **107**. In turn, high-voltage pulses are required to provide a spark discharge through the concretion **107**. Therefore, such high-voltage pulses should have energy sufficient for fragmentation of the concretion into smaller pieces. The nanosecond electro-pulse generator **104** and the laser radiation generator **105** are powered by corresponding power supply units **102** and **103**, correspondingly.

The device **10** also includes a monitoring and control system **101** configured for controlling, monitoring and selecting operating parameters of the device **10**. The monitoring and control system **101** is configured also for switching operation of the device **10** from providing laser radiation to providing nanosecond electric pulses for transferring the energy of laser radiation and nanosecond electric pulses to the concretion **107**, correspondingly. In operation, the monitoring and control system **101** provides monitoring and controlling of the device **10** that allows setting the required parameters required for the operation of both the laser radiation generator **105** of the optical energy source **11** and the nanosecond high-voltage generator **104** of the electrical energy source **12**.

The monitoring and control system **101** performs supervision of operation of the laser radiation generator **105** and the nanosecond high-voltage generator **104**, monitoring the values of the laser radiation pulse parameters and the nanosecond electric pulses along with other operating parameters for optimum implementation of the method for breaking organo-mineral concretions into smaller pieces. Optimum implementation is achieved through combining the operations of the laser radiation generator **105** and the nanosecond electro-pulse generator **104** to transfer energy to the concretion **107** for its fragmentation upon application of optic and electric energy pulses.

According to an embodiment, the device **10** can include a manipulation system (not shown in **Fig. 1**) configured for manipulating the combined probe **106**.

According to an embodiment, treatment of an organo-mineral concretion with the combined probe **106** includes pre-treatment of a surface of the concretion with a laser radiation field that is followed by fragmentation of the concretion by applying the nanosecond electro-pulse lithotripsy technique. Thus, the device **10** combines the generation of a laser radiation field and nanosecond electric pulses in order to implement fragmentation of organo-mineral concretions.

According to another embodiment, fragmentation of a concretion begins with treatment by the nanosecond electro-pulse lithotripsy technique so as to apply electric spark discharge through the concretion to form preliminary defects in the calculus. The electric spark discharge treatment is then followed by formation of further external damage by using the laser radiation field. Finally, fragmentation culminates in secondary application of the nanosecond electro-pulse lithotripsy technique.

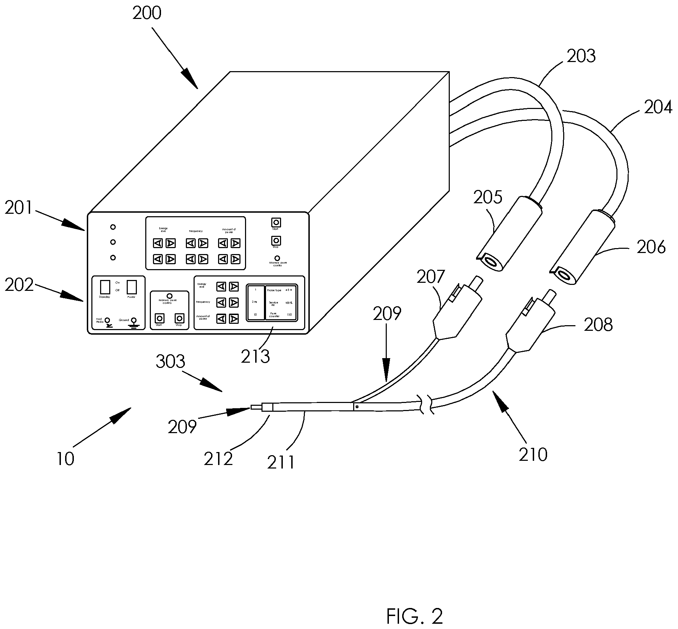

**Fig. 2** illustrates a schematic view of the device **10** for breaking a concretion in a body into smaller pieces, according to one embodiment of the present invention.

Referring to **Fig. 1** **and** **Fig. 2** together, the device **10** includes a housing module **200** for housing the monitoring and control system **101**, optical energy source **11** and an electrical energy source **12**. The device **10** also includes a nanosecond electro-pulse generator cable **204** electrically coupled to a nanosecond electro-pulse lithotripter probe **210** and a laser waveguide **203** optically coupled to a laser waveguide probe **209**. The lithotripter probe cable **204** and the laser waveguide **203** are configured for transferring energy from the generators of nanosecond pulses and the laser radiation field to the concretion (not shown) to be fragmented. The housing module **200** also includes monitoring screens **213**, and optical and electrical pulse parameter selectors **201** and **202,** which allow the operator to supervise the lithotripsy process and monitor the operating parameters for both laser treatment and electro-impulse lithotripsy treatment.

During operation of the device **10**, the level of the optical pulse energy and electrical pulse energy can be monitored and controlled; the number of pulses delivered, the frequency of the pulses, the number of pulses in each series of energy application, and the cumulative energy delivered to the calculus by each part of the device, and the power of the delivered pulses, can also be monitored. It should be understood that there can also be other parameters of the treatment process that can be controlled by the monitoring and control system **101** and displayed on the screens **213**.

Control signals from the monitoring and control system **101** are transferred to the power supply units **102**, **103** to activate operation of the laser radiation generator **105** and the nanosecond electric pulse generator **104**. Nanosecond high-voltage pulses and laser radiation pulses are relayed to the combined probe **106**, which includes both a nanosecond electro-pulse lithotripter probe **210** and a laser waveguide probe **209**. The combined probe (**106** in **Fig. 1**) is indicated by a reference numeral **211** in **Fig. 2**.

A head **212** of the combined probe **211** at a distal end **303** includes electrodes (not shown) of the nanosecond electro-pulse lithotripter probe **210** and one or more laser fibers of the laser waveguide probe **209**.

The lithotripter electrodes and the laser fiber(s) can be moved independently relative to one another, thus enabling two types of treatments, for example, a preliminary treatment of a concretion with laser radiation field and a further fragmentation of the concretion by using the lithotripter probe sequentially and independently of one another.

According to an embodiment, the monitoring and control system **101** of the device **10** includes a laser radiation parameter selector (indicated by a reference numeral **201**) and a nanosecond electro-pulse lithotripter parameter selector (indicated by a reference numeral **202**). In operation, the pulse parameter selectors **201** and **202** coordinate operation of the monitoring and control unit, that in turn coordinates operation of the optical energy source **11** and the electrical energy source **12**.The nanosecond electro-pulse generator **104** and the laser radiation generator **105** receive corresponding control signals from the monitoring and control system **101**. In turn, the monitoring and control system **101** sets the values of the required parameters of laser radiation energy and nanosecond high-voltage pulses using the pulse parameter selectors **201** and **202**. The electro-pulse energy and the laser light energy from the generators **104** and **105**, correspondingly, is transferred through the power transmission lines to the combined probe **106**.

According to an embodiment, a laser waveguide **203** is used in order to transmit laser radiation, whereas an electro-pulse generator cable **204** is used in order to transmit nanosecond high-voltage electric pulses. The cable **204** can, for example, include a coaxial cable and/or a twisted pair two-wire. The laser waveguide **203** and the electro-pulse generator cable **204** are flexible, elastic elements that can be moved independently, relative to one another.

The laser waveguide **203** and the electro-pulse generator cable **204** have corresponding connectors, such as a laser waveguide connector **205**, and a nanosecond electro-pulse cable connector **206**. The connectors **205** and **206**, in turn, are linked, correspondingly, to a waveguide connector **207** associated with the laser waveguide probe **209** and to a cable connector **208** associated with the nanosecond electro-pulse lithotripter probe **210** at their proximal ends. It should be noted that in the description and claims that follow, the terms "proximal" and "distal" are used with reference to the operator of the medical device.

The connectors **205-208** are matched correspondingly to one another to avoid losses during signal transmission. Thus, for transferring nanosecond electric pulses, the connectors **206** and **208** should have at least the same wave impedance, also matched to the cable **203** and the cable (not shown) of the nanosecond electro-pulse lithotripter probe **210**. By the same token, the connectors **205** and **207**, the waveguide **203** and the laser fiber(s) **213** of the laser waveguide probe **209** should have the same transferring properties for the selected wavelength.

As will be described hereinbelow in detail, the laser fiber(s) of the laser waveguide probe **209** and the cable of the nanosecond electro-pulse probe **210** are combined together at their distal ends under a common outer sheath (not shown) to form the combined probe **211**. The nanosecond electro-pulse probe **210** is equipped with an operating head **212** including potential and ground electrodes (not shown) coupled to the cable of the nanosecond electro-pulse probe **210**.

For example, in operation, during urological procedures, the combined probe **211** of the device **10** can be placed into a urological endoscope (not shown). The distal end **213** of the laser fiber(s) of the laser waveguide probe **209** or the electrodes of the operating head **212** nanosecond electro-pulse probe nanosecond arranged at the distal end of the nanosecond electro-pulse probe **210** can be brought to the concretion in order to apply fragmentation energy thereto.

The total length of the combined probe **211** together with the laser waveguide probe **209** and the electro-pulse lithotripter probe **210** can, for example, be within 400 mm to 2500 mm, however, depending on the clinical requirements, these values can be varied upward or downward. A length of the operating head **212** at the distal end of the nanosecond electro-pulse lithotripter probe **210** can, for example, be in the range of 5mm to 20 mm, and the outer diameter of the distal end of the combined probe can, for example, be from 0.6 mm to 5 mm, but these values can be also changed depending on the specific clinical application.

According to one embodiment, the combined probe **211** is made as a fixed unit including laser waveguide probe **209** and the electro-pulse lithotripter probe **210**, which are immovable relative to one another.

According to another embodiment, the laser waveguide probe **209** can be movable relative to the electro-pulse lithotripter probe **210**.

According to one embodiment, in operation, the start of fragmentation of the calculus or other concretion is carried out with application of laser energy emitted from the laser fiber of the laser waveguide probe **209** to the concretion surface in order to generate initial surface defects thereon. After application of laser energy, the operating head **212** of the nanosecond electro-pulse lithotripter probe **210** can be brought to the surface of the concretion damaged by the laser radiation to apply electric spark discharge through the concretion, that completes fragmentation of the concretion.

According to another embodiment, the fragmentation of a calculus starts from the treatment by the nanosecond electro-pulse lithotripter probe **210** so as to apply electric spark discharge through the concretion to form preliminary defects in the calculus. The electric spark discharge treatment is then followed by formation of further external damage by using the laser radiation emitted from the laser fiber(s) of the laser waveguide probe **209**, and then the fragmentation culminates in secondary application of the nanosecond electro-pulse lithotripter probe **210**.

According to an embodiment, the monitoring and control system **101** can also be programmed for estimation of the operational life of the device **10** to provide information on the remaining operational life of the lithotripter probe and the laser waveguide probe. When desired, information about the amount of energy that has passed through the lithotripter probe and through the laser waveguide probe can also be provided.

According to an embodiment, the monitoring and control system **101** is also configured for monitoring the operational life of the electro-pulse lithotripter probe and the laser waveguide, and notifies the operator in advance of expiration of the operating life.

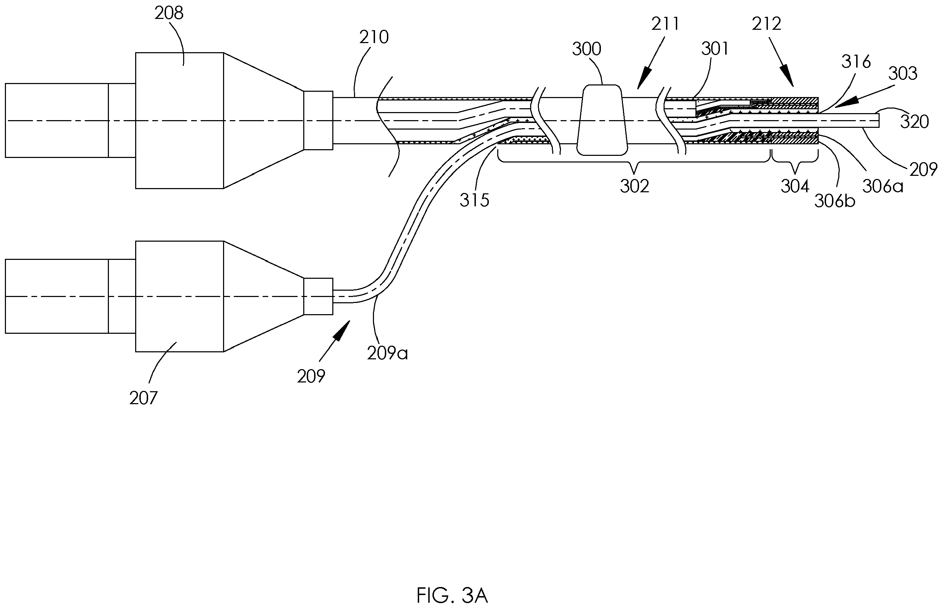

Referring to **Figs. 3A**, a schematic side partially cross-sectional view of the combined probe **211** of the medical device **10** for breaking a concretion into smaller pieces is illustrated, according to one embodiment of the present invention.

The combined probe **211** includes the laser waveguide probe **209** and the nanosecond electro-pulse lithotripter probe **210** connected to the generators (**11** and **12** in **Fig. 1**) of laser radiation and high-voltage nanosecond pulses, correspondingly via connectors **207** and **208.** The combined probe **211** also includes an external sheath **301** at a distal portion of the combined probe **211.** The distal portion of combined probe **211** has zones **302** and **304**, and a distal end **303**. The external sheath **301** of the combined probe **211** can be made of various materials, such as polyimide, braided reinforced polyimide, polyvinyl chloride, silicone rubber, nitinol, nylon, polyurethane, polyethylene terephthalate (PETE) latex, and thermoplastic elastomers, etc.

According to the embodiment shown in **Fig. 3A**, the laser waveguide probe **209** includes a laser fiber **209a** arranged along the longitudinal axis of the combined probe **211**. A distal portion **320** of the laser fiber **209** is located coaxially with the operating head **212** of the electro-pulse lithotripter probe **210** within the external sheath **301** of the combined probe **211**. The laser fiber **209a** is introduced in the external sheath **301** into a lumen **316** of the combined probe **211** through an enter opening **315**, and is then located in zones **302**, **304** up to the distal end **303** of the combined probe **211**. In operation, the laser fiber **209** can exit out of the external sheath **301** from the distal end **303** towards the concretion.

The enter opening **315** can, for example, be located 15 cm to 120 cm away from the distal end **303** of the combined probe **211**. However, it should be understood that this distance can be varied in any direction, depending on the clinical application of this device.

The operating head **212** of the electro-pulse lithotripter probe **210** is located in the zone **304** and includes electrodes (**306a** and **306b**). Thus, the laser fiber and the electrodes of the nanosecond are arranged in the combined probe **211** under the common external sheath **301**.

According to the embodiment shown in **Fig. 3A**, the combined probe **211** also includes a manipulator **300** located in the zone **302**. The manipulator **300** is configured to permit movement of the laser fiber **209a** and the operation head **212** of the electro-pulse lithotripter probe **210** independently of one another. It should be understood that the manipulator **300** is designed to simplify the procedure of movement of the fiber **209a** and the operation head **212** relative to one another, but such movement can also be carried out without such a special manipulator. The operation can be carried out by moving the laser fiber **209a** inside of the lumen **316** which is located coaxially in the probe **210** or through the movement of the head **212** of the probe **210** itself towards the concretion.

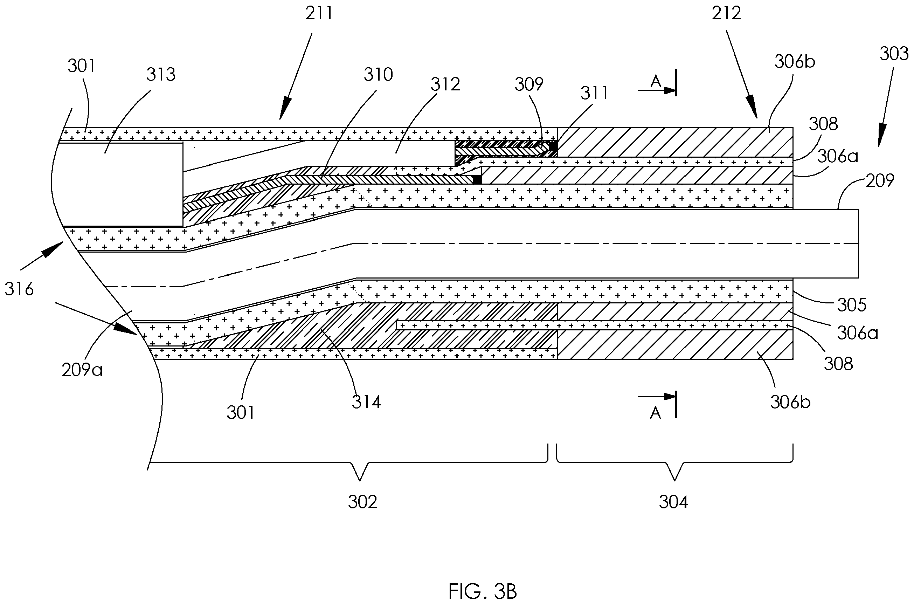

**Fig. 3B** shows a detailed enlarged view of the combined probe **211 of** **Fig. 3A**. As described above, the laser fiber **209a** enters into the electro-pulse lithotripter probe **210** in the enter opening (**315** in **Fig. 3A**) and is located within the operating head **212** of the electro-pulse lithotripter probe along the longitudinal axis of the combined probe **211**. Thus, the electrodes **306a** and **306b** surround the laser fiber **209a**.

While moving along the lumen **316**, the laser fiber **209a** can leave the distal end **303** of the combined probe **211** and can be brought into contact with the concretion (not shown) to irradiate its surface and create preliminary cracks. Then, the laser fiber **209a** can be retracted into the probe **211**. After treatment of the concretion with laser radiation, the electrodes **306a** and **306b** of the nanosecond electro-pulse lithotripter probe head **212** can be brought into contact with the concretion for its fragmentation.

According to one embodiment, the laser fiber **209a** is separated from the operating elements of the nanosecond electro-pulse lithotripter probe head **212** by the lumen **316** which forms a dielectric insulator layer **305** between the electrodes **306a** and **306b** and the fiber **209a** , thus preventing creation of potentials on the laser fiber. When desired, the lumen **316** can be filled with a dedicated insulation material to form the insulation layer **305**. The insulation layer **305** can be made of various dielectric elastic materials, such as polyvinyl chloride, rubber, polyimide, braided reinforced polyimide, silicone rubber, nitinol, nylon, polyurethane, polyethylene terephthalate (PETE) latex, and thermoplastic elastomers, etc.

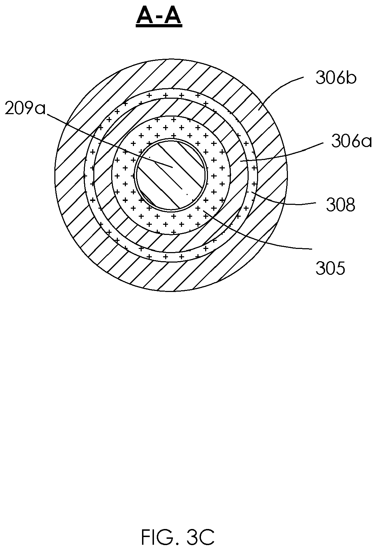

Referring to **Fig. 3C**, the described embodiment of the combined probe **211** is also represented on the A-A cross-section. The electrodes **306a** and **306b** of the operating head **212** of the nanosecond electro-pulse lithotripter probe are located around the insulation layer **305**. The electrode **306a** (internal electrode) and the electrode **306b** (external electrode) can, for example, be formed as concentrically placed tubular bushings, separated by a tubular insulator layer **308** placed between them. The electrodes **306a** and **306b** can, for example, be made from an electrically conductive material with a relatively high conductivity and can be manufactured from metals of various groups such as, for example, steel or multicomponent alloys, preferably from stainless steel or cobalt-nickel alloys. The insulator layer **308** has a high dielectric strength and is made, for example, from polyimide, ceramics, nanoceramics, etc. When desired, the insulator layer **308** can be made from several insulating bushings made from dielectric material that can be connected together with glue to increase dielectric breakdown resistance and increase erosion resistance.

During fragmentation of a concretion, the operating action includes application of laser energy to the concretion by using the laser fiber **209a** that emits laser radiation, as well as application of the electric discharge occurring between the electrodes **306a** and **306b** through the concretion.

Turning back to **Figs. 3A** **and** **3B** together, electric pulses are transferred to the electrodes **306a** and **306b** from the nanosecond electro-pulse generator (**104** in **Fig. 1**) through the cable **312**. As shown in **Figs. 3A** **and** **3B**, the coaxial cable **312** can be connected to one of the cylindrical electrodes (e.g., to the electrode **306b** that is external electrode) via its central (potential) core wire **309** and to the other cylindrical electrode **306a** via the cable braid shield **310**. It should be understood that, when desired, the connection of the cable **312** to the electrodes **306a** and **306b** can also be reversed, that is, the cable shield can be connected to the external electrode **306b**, whereas the central (potential) cable core can be connected to the electrode **306a**. Furthermore, the cable shield can be grounded or zeroed out. The electrical cable **312** has an insulation layer between the core **309** and the cable shield **310** that is made of a dielectric material having high dielectric strength, for example, a type of Teflon (polytetrafluoroethylene), etc. In making the above-mentioned connection, the core **309** of the cable **312** with its insulation and the cable braid shield **310** are pre-stripped from an external jacket **313** of the coaxial cable **312**.

Connection of the core **309** and the cable shield **310** to the electrodes **306a** and **306b** of the nanosecond electro-pulse lithotripter head **212** can be made by several methods used in bounding of electrical wires, but it is preferable that they are linked with soldering **311**. In so doing, the connection points must be spatially separated, at least at a distance no less than 2 mm to reduce the probability of breakdown at the point of the cable connection to the electrodes when high-voltage pulses are transmitted.

According to an embodiment, a twisted pair cable can also be used instead of a coaxial cable.

The voids formed during assemblage of the combined probe **211** can be filled with glue **314**. It is preferable to use high-strength glues with good dielectric properties, for example, epoxy adhesive.

The combined probe **211** is arranged such that, during the lithotripsy procedure, the laser waveguide probe **209** can be placed in various positions relative to the operating head **212** of the nanosecond electro-pulse lithotripter **210** while being moved either with the manipulator **300**, or manually. For example, the laser fiber **209a** can project over the head **212** of the nanosecond electro-pulse lithotripter, such that that its position is specific to the start of the operation when energy from the laser generator is applied through the fiber to the calculus to form surface defects. Alternatively, the laser fiber **209a** can also be located inside the nanosecond electro-pulse lithotripter head, and this position is also acceptable when the laser waveguide is not used, while the nanosecond electro-pulse lithotripter probe is only operating in order to fragment the concretion after its laser treatment.

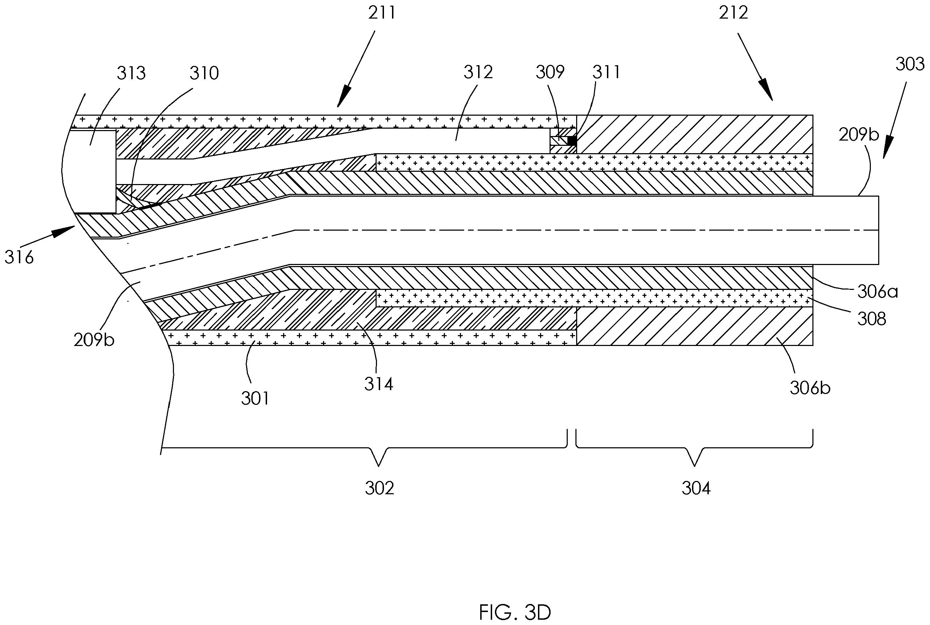

One more alternative embodiment of the combined probe **211a** is illustrated in **Fig. 3D**. The combined probe **211a** shown in **Fig. 3D** differs from the combined probe **211** shown in **Figs**. **3A-3C** by the fact that the laser fiber **209b** initially has a dielectric outer sheath (not shown) that is tightly fitted on the outer surface of the laser fiber, thus forming an integral unit with the fiber. According to this embodiment, the lumen **316** surrounding the laser fiber **209a** is filled with a conductive material. In this case, it is connected directly to one of the conductors of the coaxial cable (e.g., to the cable shield **310**, as shown in **Fig. 3D**), and thus serves as one of the electrodes (i.e. electrode 306a in **Fig. 3D**) of the nanosecond electro-pulse lithotripter.

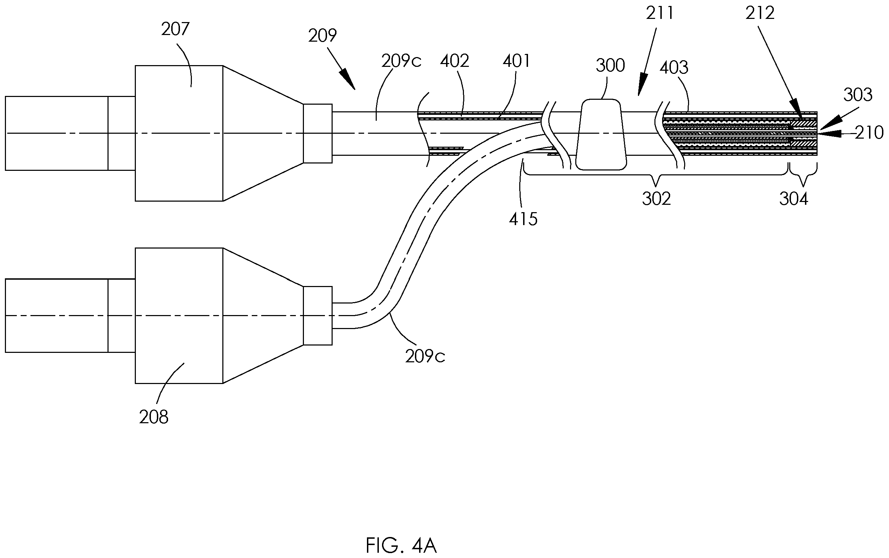

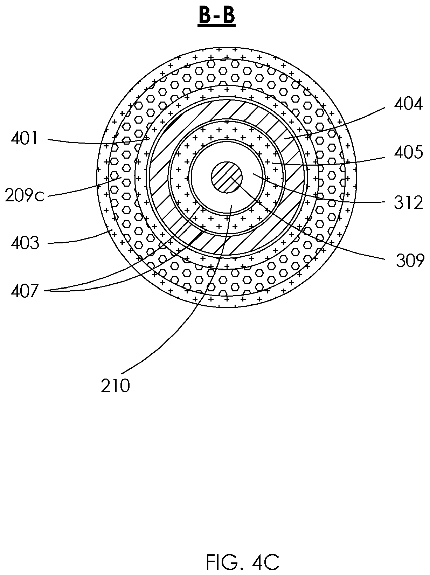

**Figs. 4A-4C** show a combined probe **211c** of the nanosecond electro-pulse lithotripter probe **210**, according to a further embodiment of the present invention. In this embodiment, the laser fiber **209a** of the laser waveguide probe **209** and the operating head **212** of the nanosecond electro-pulse lithotripter probe **210** are also located in axial alignment. However, the combined probe **211c** shown in **Figs. 4A** - **4C** differs from the combined probe **211** shown in Figs. **3A-3C** by the fact that the operating head **212** of the nanosecond electro-pulse lithotripter probe is located within a tubular laser fiber **209c**.

According to this embodiment, the laser fiber **209c** is shaped as a hollow tube, and the nanosecond electro-pulse lithotripter probe **210** the nanosecond electro-pulse lithotripter probe **210** can be introduced inside of a hollow lumen **401** of the laser fiber **209c**.

In this case, the internal wall of the tubular laser fiber **209c** serves as a wall of the lumen **401**. An external wall of the laser fiber **209c** can be surrounded by an external sheath **403** which can be an external jacket of the laser fiber **209c**.

In this embodiment, the operating head **212** of the nanosecond electro-pulse lithotripter probe **210** is introduced in the laser waveguide **209c** in an enter area **415** and is then located in zones **302**, **304**, near the distal end **303** of the combined probe **211**.

Thus, the laser waveguide probe **209** and the electro-pulse lithotripter probe **210** are aggregated in a combined probe **211c** under the common external sheath **403**.

The external sheath **403** can be made from various elastic dielectric materials. Examples of suitable materials include, but are not limited to, polyimide, polyvinyl chloride, rubber, silicone rubber, nitinol, nylon, polyurethane, polyethylene terephthalate (PETE) latex, and thermoplastic elastomers.

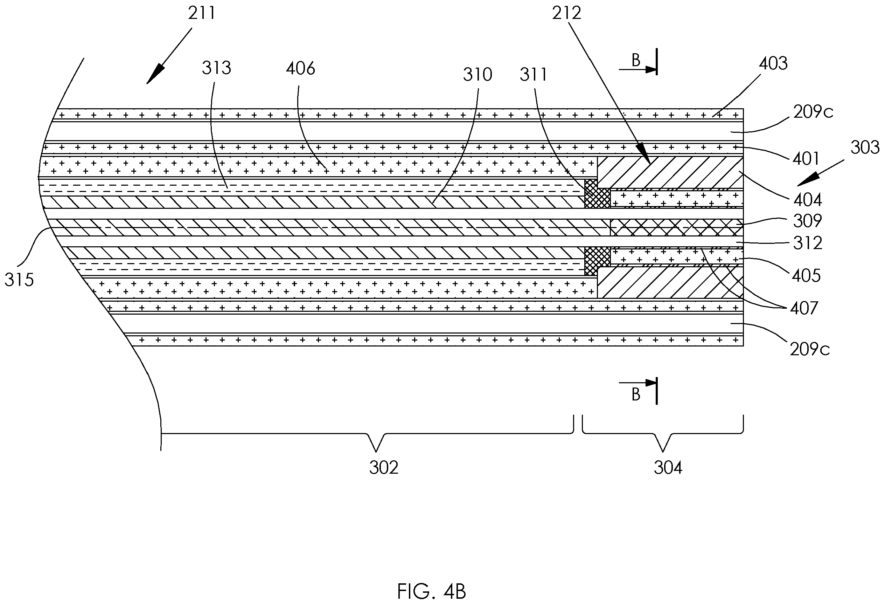

As shown in detail in **Fig. 4B**, the nanosecond electro-pulse lithotripter probe **210** includes a coaxial cable connected to a central core electrode **309** and a tubular electrode **404** of the operating head **212** which are located in the zone **304** of the combined probe **211c**. The tubular electrode **404** of the operating head **212** is in the form of a cylindrical bushing concentrically located within the lumen **401**, whereas the central core **315** of the coaxial cable of the electro-pulse lithotripter probe **210** can be connected to the electrode **309**. In this case, the electrode **404** can be connected to the cable shield **310**. Connection of the electrodes to the coaxial cable can be made by various methods used in the bounding of electric wires, such as soldering, welding etc. In particular, connection of the electrode **404** to the cable shield **310** can be made by soldering via layer **311**.

The central core electrode **309** is insulated from the tubular electrode **404** by an insulator layer **312** that can, for example, be the insulator layer of the coaxial cable. An additional insulator layer **405** having high dielectric strength (e.g., made from polyimide) can also be arranged in the zone **304** between the central core electrode **309** and a tubular electrode **404** of the operating head **212.** The insulator layer **405** can be glued to the tubular electrode **404** by a glue layer **407**. Alternatively, the insulator **405** can be manufactured from a set of tubes affixed to each other with glue in order to increase dielectric strength. The glue layer **407** must have suitable dielectric properties and mechanical strength. For example, an epoxy-based glue can be used for the glue layer **407**.

In order to impart greater rigidity to the structure during assemblage of the combined probe, a sealing layer **406** made from elastic dielectric material, e.g., from polyimide, Teflon or other polymeric material, can be arranged in the cavity **401** between an external jacket **313** of surrounding of the cable shield **310** and the internal wall the tubular fiber **209c**. In operation, using the manipulator **300** or manually, either the fiber **209c** or the lithotripter electrodes **404** and **309** can be brought into contact with the concretion independently of one another.

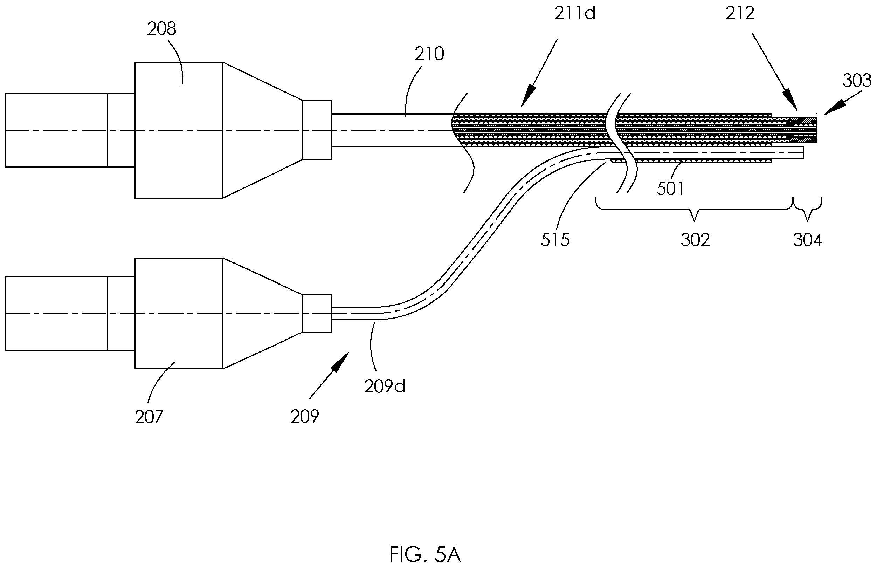

Referring to **Figs. 5A** - **5C**, a combined probe **211d** is shown, according to a further embodiment of the present invention. According to this embodiment of the combined probe **211d** differs from the combined probe shown in **Figs. 3A-3C** **and** **4A****-****4C**by the fact that a laser fiber **209d** of the laser waveguide probe **209** is arranged in parallel relation to the operating head **212** of the nanosecond electro-pulse lithotripter probe **210** under a common external sheath **501**.

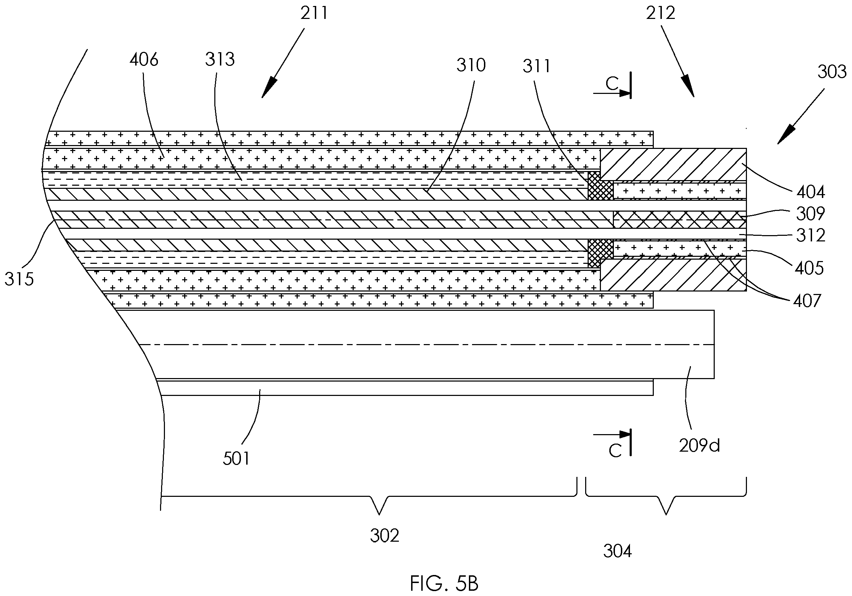

**Fig. 5B** represents a detailed enlarged view of the combined probe **211d** in zones **302** and **304**. The laser fiber **209d** is introduced in the external sheath of the combined probe **211d** in an opening **515** of the common external sheath **501**. The common external sheath **501** of the combined probe can be made of various materials including, but are not limited to polyimide, braided reinforced polyimide, polyvinyl chloride, silicone rubber, nitinol, nylon, polyurethane, polyethylene terephthalate (PETE) latex, and thermoplastic elastomers.

According to this embodiment, the nanosecond electro-pulse lithotripter probe **210** includes a coaxial cable connected to a central core electrode **309** and a tubular electrode **404** of the operating head **212** which are located in the zone **304** of the combined probe **211d**. The tubular electrode **404** of the operating head **212** is in the form of a cylindrical bushing connected to the cable shield **310**. The central core **315** of the coaxial cable of the electro-pulse lithotripter probe **210** is connected to the electrode **309**. The connection of the electrodes **309** and **404** to the coaxial cable can be made by various methods used in the bounding of electric wires, such as soldering, welding etc. In particular, the connection of the electrode **404** to the cable shield **310** can be made by soldering via the layer **311**.

The central core electrode **309** is insulated from the tubular electrode **404** by an insulator layer **312** that can, for example, be an insulator layer of the coaxial cable. An additional insulator layer **405** having high dielectric strength (e.g., made from polyimide) can also be arranged in the zone **304** between the central core electrode **309** and a tubular electrode **404** of the operating head **212**. The insulator layer **405** can be glued to the tubular electrode **404** by a glue layer **407**. Alternatively, the insulator **405** can be manufactured from a set of tubes affixed to each other with glue in order to increase dielectric strength. The glue layer **407** must offer good dielectric properties and mechanical strength. An epoxy-based glue can be used for the glue layer **407**.

The laser fiber **209d** of the laser waveguide probe **209** is arranged in a parallel relation to the lithotripter electrodes **404** and **309** of the operating head **212** under the common external sheath **501**. In operation, either the fiber **209d** or the lithotripter electrodes **404** and **309** can be brought into contact with the concretion independently of one another.

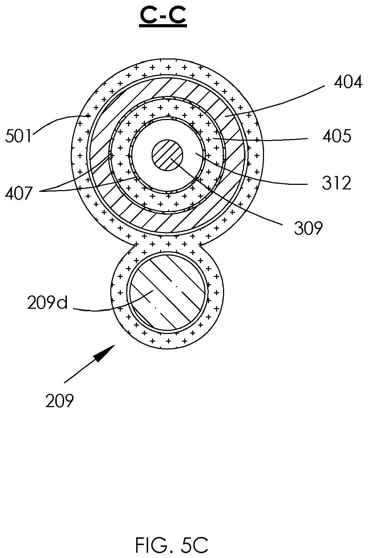

A cross-section of the combined probe **211d** along the C-C line is presented in **Fig. 5C**.

According to another aspect of the present invention, there is provided a novel method for breaking a concretion into smaller pieces by using the combined probe of the present invention. The method includes generating laser radiation field having energy sufficient to form defects on a surface of the concretion, and applying the laser radiation field to the surface of the concretion for treating the surface with a laser radiation field to create a defect. The method also includes generating nanosecond high-voltage pulses having energy sufficient to create a spark discharge through the concretion and applying the spark discharge to the concretion in the area of the defect created by the laser radiation field for fragmenting the concretion into smaller pieces.

According to an embodiment, treatment of an organo-mineral concretion with the combined probe includes pre-treatment of a surface of the concretion with a laser radiation field in order to generate initial surface defects thereon. The laser radiation treatment is followed by fragmentation of the concretion by applying the nanosecond electro-pulse lithotripsy technique.

According to another embodiment, fragmentation of a concretion begins with treatment by the nanosecond electro-pulse lithotripsy technique so as to apply electric spark discharge through the concretion to form preliminary defects in the concretion. The electric spark discharge treatment is then followed by formation of further external damage by using a laser radiation field. Finally, fragmentation culminates in secondary application of the nanosecond electro-pulse lithotripsy technique.

It was found that wavelength of the laser radiation may be in the ultraviolet, visible, or infrared range of the spectrum. However, in terms of safety of laser radiation, wavelengths in the range of 0.94 µm to 10.6 µm are preferable.

A total cumulative energy during laser surface treatment can, for example, be in the range of a few Joules to several thousand Joules, and preferably in the range of 15 Joules to 250 Joules.

According to one embodiment, the laser radiation field is a continuous laser radiation field.

According to another embodiment, the laser radiation field is a pulsed laser radiation field. In this case, a duration of the pulses (pulse width) of the pulsed laser radiation can be in the range of 0.1 ms to 60 ms, a pulse frequency can be in the range of 1 Hz to 30 Hz and a pulse energy in the is in the range of 0.3 Joule to 5 Joules. A power of the laser radiation field can be in the range of 0.5 W to 40 W.

According to one embodiment, a duration of the high-voltage nanosecond pulses generated by the electro-pulse lithotripter probe and applied to the concretion following laser treatment can be in the range of 100 nanoseconds to 1000 nanoseconds with a pulse rise time (pulse front) in the range of 1 ns to 50 nanoseconds. A magnitude of the pulses can be in the range of 5kV to 20 kV. An energy of the high-voltage nanosecond pulses can be in the range of 0.05 Joule to 10 Joules, preferably in the range of 0.1 Joule to 2 Joules. A single high-voltage nanosecond pulse or a train of high-voltage nanosecond pulses can be applied to the concretion. When a train of high-voltage nanosecond pulses is used, frequency of the pulses (pulse rate) can, for example, be in the range of 1Hz to 30 Hz, and preferably in the range of 3 Hz to 20 Hz.

As described above, preliminary laser treatment of a concretion surface provides defects on the concretion surface. A further treatment of the concretion by application of the nanosecond electro-pulse lithotripter (NPL) technique to the locations with the laser-generated defects provides electric discharge and breakdown through the bulk of the concretion, resulting in fragmentation of the calculus. If the concretion is fragmented into several relatively large pieces, then these large concretion pieces may again be treated with a laser radiation field and spark discharge through the concretions, if required. Thus, the treatment may run sequentially, as long as required, until the concretion has completely disintegrated.

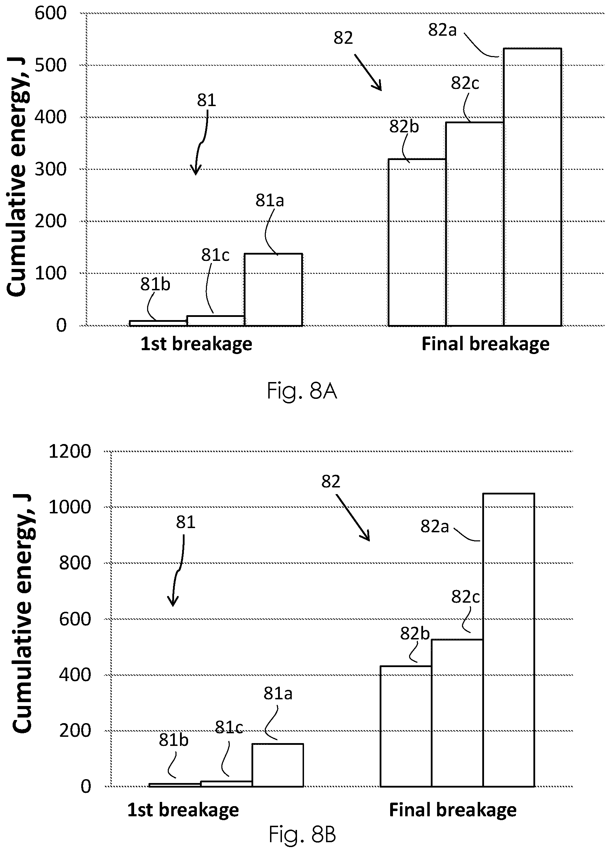

**Figs. 8A and 8B** show examples of experimental data for the cumulative energy required for a first breakage of a concretion (the experimental data are indicated by a reference numeral **81**), and the experimental data for the cumulative energy required for total disintegration of the stone pieces which remain after the first breakage (the experimental data are indicated by a reference numeral **82**). The experimental data are presented for the cases when only a NPL treatment was applied (the experimental data are indicated by reference numerals **81a and 82a**), and when a NPL treatment was applied after the preliminary laser treatment with a laser radiation field (**81b**, **81c and 82b, 82c**). The results are presented for various types of lasers.

Specifically, **Fig. 8A and 8B** corresponds to the treatment with diode laser and NPL probe (the experimental data are indicated by reference numerals **81b and 82b**); and to the treatment with Ho:YAG laser and NPL probe (the experimental data are indicated by reference numerals **81c and 82c**). **Fig. 8A** shows examples of experimental data for the treatment with 600 µm laser fiber of an Ho:YAG laser, and 4.5 Fr NPL probe, whereas **Fig. 8B** shows examples of experimental data for the treatment with 365 µm laser fiber of an Ho:YAG laser, and 3.6 Fr NPL probe. The same diode laser with 365 µm laser fiber was used for the experiments shown in **Figs. 8A and 8B**.

The experimental data show that energy consumption, and thus the time to complete fragmentation of a concretion, drops significantly when the combined treatment is used, as compared to the use of each method separately. Furthermore, it was found that the amount of reduction of cumulative energy spent for the first breakage of the concretion is not significantly dependent on the type of the laser used for treating the concretion surface (i.e. the difference is about twice the amount). However, the amount of reduction of the cumulative energy required for the first breakage by the combined technique (i.e., NPL treatment after laser treatment) is reduced by about an order of magnitude as compared to the energy required for the first breakage when only the NPL treatment was applied. Moreover, there also occurs a substantial reduction (by up to several times) of the cumulative energy and, thus, the time it takes for the stone to disintegrate completely, which is of vital importance in clinical practice.

Therefore, the initial use of laser radiation to irradiate the calculous surface, with a further fragmentation of the calculus accomplished by using the NPL device is an efficient method that makes it possible to substantially reduce the total cumulative energy and the time required for the fragmentation of various size and dense calculi.

As such, those skilled in the art to which the present invention pertains, can appreciate that while the present invention has been described in terms of preferred embodiments, the concept upon which this disclosure is based may readily be utilized as a basis for the designing of other structures and processes for carrying out the several purposes of the present invention.

It should be understood that the medical device of the present invention is not limited to medical treatment of a human body. It can be successfully employed for medical treatments of animals as well.

Moreover, the present invention is not limited to medical procedures, and may be used to shatter and extract any type of article from a wide range of inaccessible locations, such as inside a pipe or tube (for example, the waste outlet of a domestic sink) or inside a chamber within a large piece of machinery which would be difficult to dismantle.

Also, it is to be understood that the phraseology and terminology employed herein are for the purpose of description and should not be regarded as limiting.

It is important, therefore, that the scope of the invention is not construed as being limited by the illustrative embodiments set forth herein. Other variations are possible within the scope of the present invention as defined in the appended claims. Other combinations and sub-combinations of features, functions, elements and/or properties may be claimed through amendment of the present claims or presentation of new claims in this or a related application. Such amended or new claims, whether they are directed to different combinations or directed to the same combinations, whether different, broader, narrower or equal in scope to the original claims, are also regarded as included within the subject matter of the present description.

## Claims

1. A medical device for breaking an organo-mineral concretion into smaller pieces, comprising:
a combined probe including a laser waveguide probe and a nanosecond electro-pulse lithotripter probe;
an optical energy source coupled to the laser waveguide probe and configured to generate a laser radiation field having energy sufficient to form a defect on a surface of the organo-mineral concretion when the laser waveguide probe is applied to the organo-mineral concretion; and
an electrical energy source coupled to the nanosecond electro-pulse lithotripter probe and configured to generate high-voltage nanosecond electro-pulses having energy sufficient to break the organo-mineral concretion by providing a spark electrical discharge through the organo-mineral concretion when the nanosecond electro-pulse lithotripter probe is applied to the organo-mineral concretion; and
a monitoring and control system configured for monitoring operation parameters and controlling operation of the device by switching operation of the device from the activating of the laser waveguide probe for generating laser radiation to the activating of the nanosecond electro-pulse lithotripter probe for generating nanosecond electric pulses.

2. The medical device of claim 1, wherein the laser waveguide probe includes at least one laser fiber for providing the laser radiation to the organo-mineral concretion, and wherein the nanosecond electro-pulse lithotripter probe includes an operating head configured to provide spark electrical discharge through the organo-mineral concretion.

3. The medical device of claim 2, wherein a distal portion of said at least one laser fiber is arranged coaxially with the operating head of the nanosecond electro-pulse lithotripter probe.

4. The medical device of claim 3, wherein said at least one laser fiber is arranged along the longitudinal axis of the combined probe, and wherein the operating head includes lithotripter electrodes formed as concentrically placed tubular bushings surrounding the laser fiber.

5. The medical device of claim 3, wherein said at least one laser fiber has a tubular shape, and the operating head is arranged within the laser fiber.

6. The medical device of claim 3, wherein the operating head of the electro-pulse lithotripter and the laser fiber are movable independently of one another.

7. The medical device of claim 1, wherein the combined probe includes an external sheath surrounding the laser waveguide probe and a nanosecond electro-pulse lithotripter probe.

8. The medical device of claim 2, wherein said at least one laser fiber of the laser waveguide probe is arranged in parallel relation to the operating head of the nanosecond electro-pulse lithotripter probe.

9. The medical device of claim 2, wherein the range of laser wavelengths of the laser radiation is in the range of 0.94 µm - 10.6 µm.

10. The medical device of claim 2, wherein the laser radiation is continuous laser radiation.

11. The medical device of claim 2, wherein the laser radiation is pulsed laser radiation.

12. The medical device of claim 11, wherein a duration of pulses of the pulsed laser radiation is in the range of 0.1 ms to 60 ms, with a pulse frequency in the range of 1 Hz to 30 Hz and power in the range of 0.5 W to 40 W.

13. The medical device of claim 12, wherein an energy in the pulse is in the range of 0.3 Joule to 5 Joules.

14. The medical device of claim 2, wherein an amplitude of the electro-pulses of the electro-pulse lithotripter probe is in the range of 5 kV to 20 kV, and energy in the pulse is in the range of 0.1 Joule to 2 Joules.

15. The medical device of claim 2, wherein the electro-pulses of the electro-pulse lithotripter probe include a train of high-voltage nanosecond electro-pulses having a frequency in the range of 3 Hz to 20 Hz.

## Patentansprüche

1. Medizinische Vorrichtung zum Zerbrechen eines organo-mineralischen Konkrements in kleinere Stücke, umfassend:
eine kombinierte Sonde mit einer Laser-Wellenleitersonde und einer Nanosekunde-Elektroimpuls-Lithotriptorsonde;
eine optische Energiequelle, die mit der Laser-Wellenleitersonde verbunden ist und dafür ausgelegt ist, ein Laserstrahlungsfeld zu erzeugen mit Energie, die ausreichend ist, um einen Defekt auf einer Oberfläche des organo-mineralischen Konkrements zu bilden, wenn die Laser-Wellenleitersonde auf das organo-mineralische Konkrement aufgetragen wird; und
eine elektrische Energiequelle, die mit der Nanosekunde-Elektroimpuls-Lithotriptorsonde verbunden ist und dafür ausgelegt ist, Hochspannungs-Nanosekunde-Elektroimpulse zu erzeugen mit Energie, die ausreichend ist, um das organo-mineralische Konkrement durch Bereitstellung einer elektrischen Funkenentladung durch das organo-mineralische Konkrement zu zerbrechen, wenn die Nanosekunde-Elektroimpuls-Lithotriptorsonde auf das organo-mineralische Konkrement aufgetragen wird; und
ein Überwachungs- und Steuerungssystem, das dafür ausgelegt ist, Betriebsparameter zu überwachen und den Betrieb der Vorrichtung durch Schalten des Betriebs der Vorrichtung von einer Aktivierung der Laser-Wellenleitersonde zum Erzeugen von Laserstrahlung zu einer Aktivierung der Nanosekunde-Elektroimpuls-Lithotriptorsonde zum Erzeugen von Nanosekunde-Elektroimpulse zu steuern.

2. Medizinische Vorrichtung nach Anspruch 1, wobei die Laser-Wellenleitersonde mindestens eine Laserfaser zur Bereitstellung der Laserstrahlung zum organo-mineralischen Konkrement aufweist, und wobei die Nanosekunde-Elektroimpuls-Lithotriptorsonde einen Arbeitskopf aufweist, der dafür eingerichtet ist, eine elektrische Funkenentladung durch das organo-mineralische Konkrement bereitzustellen.

3. Medizinische Vorrichtung nach Anspruch 2, wobei ein distaler Teil der mindestens einen Laserfaser koaxial zum Arbeitskopf der Nanosekunde-Elektroimpuls-Lithotriptorsonde angeordnet ist.

4. Medizinische Vorrichtung nach Anspruch 3, wobei die mindestens eine Laserfaser entlang der Längsachse der kombinierten Sonde angeordnet ist, und wobei der Arbeitskopf Lithotriptorelektroden aufweist, die als konzentrisch angeordnete, rohrförmige, die Laserfaser umgebende Buchsen gebildet sind.

5. Medizinische Vorrichtung nach Anspruch 3, wobei die mindestens eine Laserfaser eine rohrförmige Form hat, und der Arbeitskopf in der Laserfaser angeordnet ist.

6. Medizinische Vorrichtung nach Anspruch 3, wobei der Arbeitskopf des Elektroimpuls-Lithotriptors und die Laserfaser unabhängig voneinander bewegbar sind.

7. Medizinische Vorrichtung nach Anspruch 1, wobei die kombinierte Sonde eine Außenummantelung aufweist, die die Laser-Wellenleitersonde und eine Nanosekunde-Elektroimpuls-Lithotriptorsonde umgibt.

8. Medizinische Vorrichtung nach Anspruch 2, wobei die mindestens eine Laserfaser der Laser-Wellenleitersonde parallel zum Arbeitskopf der Nanosekunde-Elektroimpuls-Lithotriptorsonde angeordnet ist.

9. Medizinische Vorrichtung nach Anspruch 2, wobei der Bereich von Laserwellenlängen der Laserstrahlung im Bereich von 0,94 µm - 10,6 µm liegt.

10. Medizinische Vorrichtung nach Anspruch 2, wobei die Laserstrahlung kontinuierliche Laserstrahlung ist.

11. Medizinische Vorrichtung nach Anspruch 2, wobei die Laserstrahlung gepulste Laserstrahlung ist.

12. Medizinische Vorrichtung nach Anspruch 11, wobei eine Dauer von Impulsen der gepulsten Laserstrahlung im Bereich von 0,1 ms bis 60 ms liegt, mit einer Impulsfrequenz im Bereich von 1 Hz bis 30 Hz und Leistung im Bereich von 0,5 W bis 40 W.

13. Medizinische Vorrichtung nach Anspruch 12, wobei eine Energie im Impuls im Bereich von 0,3 Joule bis 5 Joule ist.

14. Medizinische Vorrichtung nach Anspruch 2, wobei eine Amplitude der Elektroimpulse der Elektroimpuls-Lithotriptorsonde im Bereich von 5 kV bis 20 kV ist, und Energie im Impuls im Bereich von 0,1 Joule bis 2 Joule ist.

15. Medizinische Vorrichtung nach Anspruch 2, wobei die Elektroimpulse der Elektroimpuls-Lithotriptorsonde eine Reihe von Hochspannungs-Nanosekunde-Elektroimpulsen mit einer Frequenz im Bereich von 3 Hz bis 20 Hz aufweisen.

## Revendications

1. Dispositif médical pour casser une concrétion organo-minérale en morceaux inférieurs, comprenant :
une sonde combinée comprenant une sonde à guide d'ondes laser et une sonde de lithotriteur à électro-impulsions de nanoseconde ;
une source d'énergie optique couplée à la sonde à guide d'ondes laser et configurée pour générer un champ de rayonnement laser ayant une énergie suffisante pour former un défaut sur une surface de la concrétion organo-minérale lorsque la sonde à guide d'ondes laser est appliquée à la concrétion organo-minérale ; et
une source d'énergie électrique couplée à la sonde de lithotriteur à électro-impulsions de nanoseconde et configurée pour générer des électro-impulsions de nanoseconde à haute tension ayant une énergie suffisante pour casser la concrétion organo-minérale en fournissant une décharge électrique à étincelles à travers la concrétion organo-minérale lorsque la sonde de lithotriteur à électro-impulsions de nanoseconde est appliquée à la concrétion organo-minérale ; et
un système de commande et de surveillance configuré pour surveiller les paramètres de fonctionnement et commander le fonctionnement du dispositif en commutant le fonctionnement du dispositif depuis l'actionnement de la sonde à guide d'ondes laser pour générer un rayonnement laser vers l'actionnement de la sonde de lithotriteur à électro-impulsions de nanoseconde pour générer des impulsions électriques de nanoseconde.

2. Dispositif médical selon la revendication 1, dans lequel la sonde à guide d'ondes laser comprend au moins une fibre laser pour fournir le rayonnement laser à la concrétion organo-minérale, et dans lequel la sonde de lithotriteur à électro-impulsions de nanoseconde comprend une tête de fonctionnement configurée pour fournir une décharge électrique par étincelle à travers la concrétion organo-minérale.

3. Dispositif médical selon la revendication 2, dans lequel une partie distale de ladite au moins une fibre laser est agencée coaxialement avec la tête de fonctionnement de la sonde de lithotriteur à électro-impulsions de nanoseconde.

4. Dispositif médical selon la revendication 3, dans lequel ladite au moins une fibre laser est agencée le long de l'axe longitudinal de la sonde combinée, et dans lequel la tête de fonctionnement comprend des électrodes de lithotripteur formées comme des bagues tubulaires placées concentriquement et entourant la fibre laser.

5. Dispositif médical selon la revendication 3, dans lequel ladite au moins une fibre laser a une forme tubulaire, et la tête de fonctionnement est agencée dans la fibre laser.

6. Dispositif médical selon la revendication 3, dans lequel la tête de fonctionnement du lithotripteur à électro-impulsions et la fibre laser sont mobiles indépendamment l'une de l'autre.

7. Dispositif médical selon la revendication 1, dans lequel la sonde combinée comprend une gaine externe entourant la sonde à guide d'ondes laser et une sonde de lithotriteur à électro-impulsions de nanoseconde

8. Dispositif médical selon la revendication 2, dans lequel ladite au moins une fibre laser de la sonde à guide d'ondes laser est agencée en parallèle avec la tête de fonctionnement de la sonde de lithotriteur à électro-impulsions de nanoseconde.

9. Dispositif médical selon la revendication 2, dans lequel la plage de longueurs d'onde du laser est dans la plage de 0,94 µm à 10,6 µm.

10. Dispositif médical selon la revendication 2, dans lequel le rayonnement laser est un rayonnement laser continu.

11. Dispositif médical selon la revendication 2, dans lequel le rayonnement laser est un rayonnement laser pulsé.

12. Dispositif médical selon la revendication 11, dans lequel une durée d'impulsions du rayonnement laser puisé est dans la plage de 0,1 ms à 60 ms, avec une fréquence d'impulsion dans la plage de 1 Hz à 30 Hz et une puissance dans la plage de 0,5 W à 40 W.

13. Dispositif médical selon la revendication 12, dans lequel une énergie dans l'impulsion est dans la gamme de 0,3 Joule à 5 Joules.

14. Dispositif médical selon la revendication 2, dans lequel une amplitude des électro-impulsions de la sonde de lithotriteur à électro-impulsions est dans la plage de 5 kV à 20 kV, et l'énergie dans l'impulsion est dans la plage de 0,1 Joule à 2 Joules.

15. Dispositif médical selon la revendication 2, dans lequel les électro-impulsions de la sonde de lithotriteur à électro-impulsions comprend un train d'électro-impulsions de nanoseconde à haute tension ayant une fréquence dans la plage de 3 Hz à 20 Hz.
